# EUROPEAN PATENT SPECIFICATION

(11) **EP 3 865 559 B1**
(45) Date of publication and mention of the grant of the patent: **16.10.2024**
(21) Application number: 20157392.0
(22) Date of filing: 14.02.2020
(51) Int. Cl.: C10L 3/08, C07C 1/12, C07C 9/04, C25B 1/04, C25B 15/08, G06Q 50/06, F02B 43/10, F23D 14/28, F23D 14/32

(54) **METHOD FOR OPERATION OF AN INDUSTRIAL PLANT**
VERFAHREN ZUM BETRIEB EINER INDUSTRIEANLAGE
PROCÉDÉ DE FONCTIONNEMENT D'UNE INSTALLATION INDUSTRIELLE

(43) Date of publication of application: 18.08.2021
(73) Proprietor: Siemens Energy Global GmbH & Co. KG, 81739 München (DE)
(72) Inventor: Kubis, Stanislav, 66482 Ricany (CZ)

(56) References cited:
- WO-A2-2013/124632
- CN-A- 106 285 802
- CN-A- 110 700 944
- DE-A1- 102017 210 324

## Description

The present invention relates to a method for operation of an industrial plant.

The reduction of carbon dioxide emitted into the atmosphere on the one hand and increasing the share of renewable on the other hand cause some problems. One of the problems is how to use or store the electricity excess from the renewable sources, since the amount of produced power cannot be effectively controlled. Due to this fact, there is energy generated during times when there is low or no consumption for it. This surplus of electricity is currently accumulated in different kinds of energy accumulators such as pumped water storage plant, underground pneumatic accumulators, electric accumulators, etc.

Recently, new methods of energy storage have been applied at industrial scale. The electrical energy can be accumulated or stored by converting it into chemical energy of synthetic fuels such as hydrogen, methane or synthetic natural gas (SNG). In the prior art this process is referred to as "power to gas" technology.

The following documents in particular are to be regarded as prior art in relation to the present invention:
DE 10 2017 210324 A discloses an industrial plant for transforming electrical power to methane / synthetic natural gas, i.e. a Power-to-Gas plant.

CN 110 700 944 A discloses an industrial plant comprising an energy accumulator unit comprising a water electrolyser and a carbon dioxide hydro-methanation unit for the production of SNG, a power plant comprising a combustor and turbines coupled to a generator, as well as storage tanks for oxygen, carbon dioxide and water.

CN 106 285 802 A discloses an industrial plant comprising an energy accumulator unit comprising a water electrolyser and a methane production unit for the production of SNG from hydrogen and C02, a power plant comprising a turbine, as well as storage tanks for oxygen, carbon dioxide and water.

WO 2013/124632 A describe the integration with district heating in the process.

One of the emerging technologies to accumulate the electricity excess is through the production of synthetic natural gas (SNG). The technology is based on the so-called Sabatier reaction, in which synthetic natural gas and water are formed from carbon dioxide (CO₂) and hydrogen (H₂) in the presence of catalysts. In the published studies the Carbon dioxide is obtained from biogas or coal while the hydrogen is obtained by electrolysis of water. Unfortunately, both substances biogas and coal contain certain additives. These additives play the role of impurities in the Sabatier reaction and contaminate the catalyst. This contamination reduces the efficiency of the Sabatier reaction until the reaction is not effective and the reactor has to be shutdown to recover the catalyst. It is necessary to transport these reactants for the Sabatier reaction from distant sites to the synthetic natural gas production site. Hence, there are two essential disadvantages of the above referred method: on the one hand the necessity to transport carbon dioxide or oxygen from distant locations and on the other hand the impurities in the carbon dioxide caused by the fuel additives.

It is object of the present invention to provide an efficient process of accumulating the electricity surplus, which process eliminates the above-identified disadvantages.

The object of the invention is achieved by the independent claims. The dependent claims describe advantageous developments and modifications of the invention.

In accordance with the invention there is provided a method for operation of an industrial plant comprising:
- an energy accumulator unit for production of synthetic natural gas,
- a power plant unit for production of electricity,
- an oxygen tank, a carbon dioxide tank and a water tank, wherein
- in a first operation mode the energy accumulator unit is supplied with excessed electricity from the public grid in order to produce synthetic natural gas, wherein the produced synthetic natural gas is discharged in a gas network, while oxygen and water which are produced together with the synthetic natural gas are stored in the oxygen tank and the water tank correspondingly,
- in a second operation mode gas from the gas network together with oxygen from the oxygen tank and water from the water tank are used in the power plant unit to produce electricity, which is supplied to the public grid
- wherein in the second operation mode the gas supplied from the gas network is burned together with oxygen from the oxygen tank in a combustor and the combustion mixture is used to drive a turbine
- wherein water from the water tank is used to regulate the temperature in the combustor.

The essential idea of the present invention is to connect the industrial plant to the public grid which transfers easily surplus renewable energy from distant locations to the industrial plant. This surplus energy is used for the production of synthetic natural gas, which is then fed into a gas network. The gas network could be a public network used to transport the synthetic natural gas to consumers at distant sites. The gas network could be also a local network, i.e. a network serving only the industrial plant, an industrial zone or a residential area in close proximity to the industrial plant. Any type of gas network may include storage, e.g. means for underground storage, in case of excess of the synthetic natural gas production. Along with the synthetic natural gas, further oxygen and water are produced in the energy accumulator unit, which are stored locally in the industrial plant.

In the second operation mode, the oxygen and water stored on site are used for the production of electricity in the power plant unit of the industrial plant. The electricity generated in the power plant unit is fed into the public grid.

Thus, in the industrial plant surplus energy is used to produce synthetic natural gas (which is feed into a gas network) and oxygen, which oxygen later is used together with gas from the gas network to produce energy for the public grid.

In a preferred embodiment in the first operation mode the SNG is produced in a Sabatier reaction which involves the reaction of hydrogen with carbon dioxide at elevated temperatures (optimally 300-400 °C) and pressures in the presence of a catalyst to produce methane and water.

CO₂ + 4H₂ -> CH₄ + 2H₂O

The Sabatier reaction takes part in a Sabatier reactor which is part of the energy accumulator unit. Methanation is an important step in the creation of synthetic or substitute natural gas (SNG). The methane or SNG can be injected into the existing gas network, which has storage capacity of many months up to a couple of years.

In another preferred embodiment water from the water tank is used in an electrolysis reaction to produce oxygen and hydrogen, which hydrogen is used for the Sabatier reaction. Hence, the process of electrolysis of water is carried out in a preliminary step in order to create hydrogen for the Sabatier reaction. The oxygen, which is also generated in the water electrolysis, is stored in the oxygen tank of the industrial plant. In this case the water tank is not an external tank (even it might be). The water tank is accumulating and recycling the water received as product of methane burning.

In the second operation mode the gas supplied from the gas network is burned together with oxygen from the oxygen tank in a combustor and the combustion mixture (containing carbon dioxide and steam) is used to drive a turbine. The combustor can be an external burner which is separated from the turbine or a burner integrated in the turbine.

Water from the water tank is used to regulate the temperature in the combustor by spraying the water into the mixture of gas and steam coming out of the combustor.

Still preferably, a steam-gas-mixture from the turbine is at least partially condensed in a heat exchanger producing water and steam and carbon dioxide mixture, where water and carbon dioxide are stored in the water tank and the carbon dioxide tank correspondingly.

In order to increase the efficiency of the process running in the industrial plant, in a preferred embodiment the heat from the heat exchanger is used for district heating or applied in a condensing turbine so that a turbine with increased the power is used.

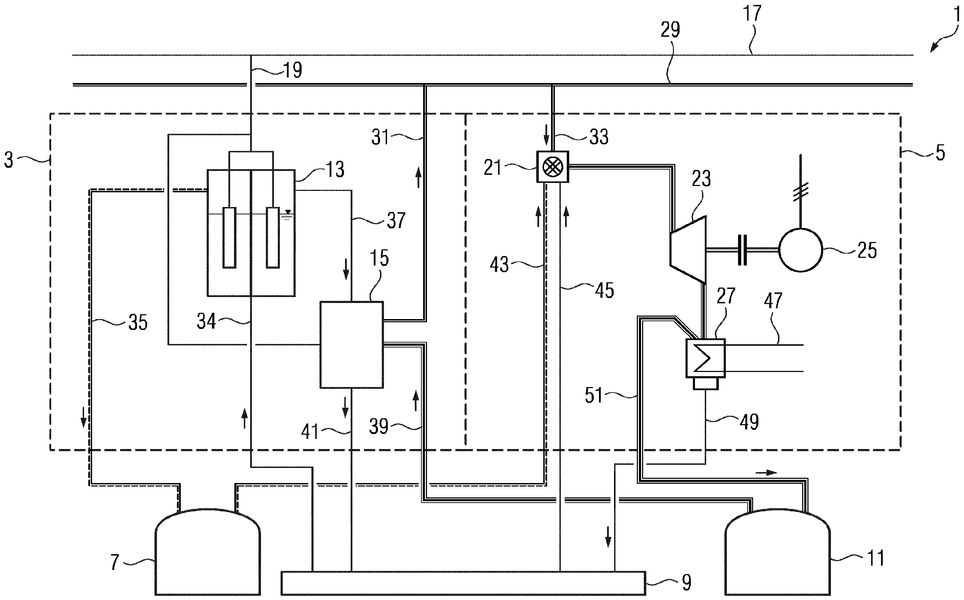

Preferably, the energy accumulator unit and the power plant unit are operated alternatingly. The energy accumulator unit is operated in times of electricity surplus, while the power plant unit is operated in order to produce supplementary electricity on demand. Alternatively, the energy accumulator unit and the power plant unit are operated simultaneously. Embodiments of the invention are now described, by way of example only, with reference to the accompanying drawing, of which the only figure shows schematically an industrial plant 1. The industrial plant 1 comprises an energy accumulator unit 3 for production of synthetic natural gas and a power plant unit 5 for production of electricity. In the power plant unit 5 additionally heat is produced. The industrial plant further comprises an oxygen tank 7, a water tank 9 and a carbon dioxide tank 11.

In the embodiment shown in the figure the energy accumulator unit 3 consists of a hydrogen electrolysis system 13 and a Sabatier reactor 15. The hydrogen electrolysis system 13 is supplied with electricity from the public grid 17 by means of a power cable 19. The Sabatier reactor 15 is connected to a gas network via a gas discharge line 31.

The power plant unit 5 consists of a combustor 21, a turbine 23, a generator 25 and a heat exchanger 27. The combustor 21 is operated with gas from the gas network 29 provided by a gas supply line 33.

In a first operation mode of the industrial plant 1, in case of renewable electricity surplus in the public grid 17, this excessed energy is accumulated or stored in the energy accumulator unit 3 in form of oxygen stored in the oxygen tank 7 and SNG produced in the Sabatier reactor 15 and supplied to the gas network via the gas discharge line 31.

Water taken from the water tank 9 via a first water feed line 34 is provided to the hydrogen electrolysis system 13, in which the water molecules are split into hydrogen and oxygen using electrical current from the public grid 17. The oxygen stream is fed via an oxygen discharge line 35 into the oxygen tank 7 for storage purposes. The hydrogen stream is provided via a hydrogen feed line 37 into the Sabatier reactor 15, in which it reacts together with carbon dioxide provided by a feed line 39 from the carbon dioxide tank 11 to produce SNG (methane). The SNG produced in the energy accumulator unit 3 is fed into the gas network 29 by means of outflow gas pipe 31 and can be potentially transported to far distant underground gas storage facilities. Another product of the Sabatier reaction is water, which is discharged in a first water discharge line 41 and stored in the water tank 9.

Hence, in the first operation mode excessed energy from the public grid is stored locally by converting it into oxygen and water and the remaining part of the excessed energy is stored in form of synthetic natural gas in the gas network or stored in distant gas storage facilities connected to the gas network.

Normally, the power plant unit 5 is shutdown during the first operation mode.

In a second operation mode gas from the gas network 29 together with oxygen from the oxygen tank 7 and water from the water tank 9 are used in the power plant unit 5 to produce electricity. Natural gas or accumulated SNG is supplied from the gas network 29 via the gas supply line 33 into the combustor 21 to generate a steam-gas-mixture. The fuel is burned there with oxygen fed from the local oxygen tank 7 via an oxygen supply line 43. The flue gas of the combustion process is a mixture of water steam and carbon dioxide and its temperature is adjusted by the amount of water from the water tank 9 via a second water feed line 45 and injected into the combustor 21. The combustion mixture is fed into a turbine 23 where it expands.

The mechanical energy from the turbine 23 is transferred to the electrical generator 25, thus generating electricity. This electrical energy is conveyed to the public electricity grid 17.

The steam-gas-mixture is discharged from the turbine 23 into a heat exchanger 27. In the heat exchanger 27 the remaining thermal energy is transferred to a coolant 47 cycle. The steam-gas-mixture is cooled down there. The water steam from the mixture condenses and is discharged through a second water discharge line 49 into the water tank 9. The incondensa-ble, clean carbon dioxide is discharged through a discharge line 51 into the local carbon dioxide tank 11.

This process, in which the locally stored energy (oxygen and water) together with SNG from the gas network are converted into electricity and heat, can be considered discharging of the energy accumulator unit 3. In this cycle, the total energy of methane, i.e. the upper heating value is employed compared with standard devices. This value is about 10% higher than the lower heating value.

Although the present invention has been described in detail with reference to the preferred embodiment, it is to be understood that the present invention is not limited by the disclosed examples, and that numerous additional modifications and variations could be made thereto by a person skilled in the art without departing from the scope of the invention.

## Claims

1. Method for operation of an industrial plant (1) comprising
- an energy accumulator unit (3) for production of synthetic natural gas,
- a power plant unit (5) for production of electricity,
- a oxygen tank (7), a carbon dioxide tank (11) and a water tank (9),
wherein
- in a first operation mode the energy accumulator unit (3) is supplied with excessed electricity from the public grid (17) in order to produce synthetic natural gas, wherein the produced synthetic natural gas is discharged in a gas network (29), while oxygen and water which are produced together with the synthetic natural gas are stored in the oxygen tank (7) and the water tank (9) correspondingly,
- in a second operation mode gas from the gas network (29) together with oxygen from the oxygen tank (7) and water from the water tank (9) are used in the power plant unit (5) to produce electricity, which is supplied to the public grid (17)
- wherein in the second operation mode the gas supplied from the gas network (29) is burned together with oxygen from the oxygen tank (7) in a combustor (21) and the combustion mixture is used to drive a turbine (23)
- wherein water from the water tank (9) is used to regulate the temperature in the combustor (21).

2. Method according to claim 1,
wherein in the first operation mode the SNG is produced in a Sabatier reaction.

3. Method according to claim 2,
wherein water from the water tank (9) is used in an electrolysis reaction to produce oxygen and hydrogen, which hydrogen is used for the Sabatier reaction.

4. Method according to any of the preceding claims,
wherein a steam-gas-mixture from the turbine (23) is at least partially condensed in a heat exchanger (27) producing water and steam and carbon dioxide mixture, where water and carbon dioxide are stored in the water tank (9) and the carbon dioxide tank (11) correspondingly.

5. Method according to any of the preceding claims,
wherein the heat from the heat exchanger (27) is used for district heating.

6. Method according to any of the preceding claims,
wherein the energy accumulator unit (3) and the power plant unit (5) are operated alternatingly.

## Patentansprüche

1. Verfahren zum Betreiben einer Industrieanlage (1), das Folgendes umfasst
- eine Energiespeichereinheit (3) zur Produktion von synthetischem Erdgas,
- eine Kraftwerkseinheit (5) zur Produktion von Elektrizität,
- einen Sauerstofftank (7), einen Kohlendioxidtank (11) und einen Wassertank (9),
wobei
- in einem ersten Betriebsmodus die Energiespeichereinheit (3) mit überschüssiger Elektrizität vom öffentlichen Netz (17) versorgt wird, um synthetisches Erdgas zu produzieren, wobei das produzierte synthetische Erdgas in ein Gasnetz (29) abgelassen wird, während Sauerstoff und Wasser, die zusammen mit dem synthetischen Erdgas produziert werden, entsprechend in dem Sauerstofftank (7) und dem Wassertank (9) gespeichert werden,
- in einem zweiten Betriebsmodus Gas vom Gasnetz (29) zusammen mit Sauerstoff aus dem Sauerstofftank (7) und Wasser aus dem Wassertank (9) in der Kraftwerkseinheit (5) verwendet werden, um Elektrizität zu produzieren, die dem öffentlichen Netz (17) zugeführt wird
- wobei im zweiten Betriebsmodus das vom Gasnetz (29) zugeführte Gas zusammen mit Sauerstoff aus dem Sauerstofftank (7) in einer Brennkammer (21) verbrannt wird und das Verbrennungsgemisch verwendet wird, um eine Turbine (23) anzutreiben
- wobei Wasser aus dem Wassertank (9) verwendet wird, um die Temperatur in der Brennkammer (21) zu regeln.

2. Verfahren nach Anspruch 1,
wobei, im ersten Betriebsmodus das SNG in einer Sabatier-Reaktion produziert wird.

3. Verfahren nach Anspruch 2,
wobei Wasser aus dem Wassertank (9) in einer Elektrolysereaktion verwendet wird, um Sauerstoff und Wasserstoff zu produzieren, wobei der Wasserstoff für die Sabatier-Reaktion verwendet wird.

4. Verfahren nach einem der vorhergehenden Ansprüche,
wobei ein Dampf-Gas-Gemisch von der Turbine (23) zumindest teilweise in einem Wärmetauscher (27) kondensiert wird, ein Gemisch aus Wasser und Dampf und Kohlendioxid produzierend, wobei Wasser und Kohlendioxid im Wassertank (9) bzw. im Kohlendioxidtank (11) gespeichert werden.

5. Verfahren nach einem der vorhergehenden Ansprüche,
wobei die Wärme vom Wärmetauscher (27) für Fernheizung verwendet wird.

6. Verfahren nach einem der vorhergehenden Ansprüche,
wobei die Energiespeichereinheit (3) und die Kraftwerkseinheit (5) alternierend betrieben werden.

## Revendications

1. Procédé pour faire fonctionner une installation (1) industrielle comprenant
- une unité (3) d'accumulation d'énergie pour la production de gaz naturel de synthèse,
- une unité (5) de centrale électrique pour produire de l'électricité,
- un réservoir (7) d'oxygène, un réservoir (11) de dioxyde de carbone et un réservoir (9) d'eau,
dans lequel
- dans un premier mode de fonctionnement, on alimente l'unité (3) d'accumulation d'énergie par un excès d'électricité provenant du réseau (17) public afin de produire du gaz naturel de synthèse, dans lequel on envoie le gaz naturel de synthèse produit dans un réseau (29) de gaz, tandis que de l'oxygène et de l'eau qui sont produits ensemble avec le gaz naturel de synthèse sont stockés dans le réservoir (7) d'oxygène et le réservoir (9) d'eau d'une manière correspondante,
- dans un deuxième mode de fonctionnement, on utilise du gaz du réseau (29) de gaz ensemble avec l'oxygène du réservoir (7) d'oxygène et de l'eau du réservoir (9) d'eau dans l'unité (5) de centrale électrique pour produire de l'électricité, qui est envoyée au réseau (17) public,
- dans lequel dans le deuxième mode de fonctionnement, on brûle le gaz fournit par le réseau (29) de gaz ensemble avec de l'oxygène du réservoir (7) d'oxygène dans une chambre de combustion (21) et on utilise le mélange de combustion pour entraîner une turbine (23),
- dans lequel on utilise l'eau du réservoir (9) d'eau pour réguler la température dans la chambre de combustion (21) .

2. Procédé suivant la revendication 1,
dans lequel dans le premier mode de fonctionnement, on produit le SNG dans une réaction de Sabatier.

3. Procédé suivant la revendication 2,
dans lequel on utilise de l'eau du réservoir (9) d'eau dans une réaction d'électrolyse pour produire de l'oxygène et de l'hydrogène, lequel hydrogène est utilisé pour la réaction de Sabatier.

4. Procédé suivant l'une quelconque des revendications précédentes,
dans lequel on condense au moins partiellement, un mélange de vapeur d'eau et de gaz provenant de la turbine (23) dans un échangeur (27) de chaleur produisant de l'eau et un mélange de vapeur et de dioxyde de carbone, l'eau et le dioxyde de carbone étant stockés dans le réservoir (9) d'eau et dans le réservoir (11) de dioxyde de carbone d'une manière correspondante.

5. Procédé suivant l'une quelconque des revendications précédentes,
dans lequel on utilise la chaleur provenant de l'échangeur (27) de chaleur pour un chauffage urbain.

6. Procédé suivant l'une quelconque des revendications précédentes,
dans lequel on fait fonctionner en alternance l'unité (3) d'accumulation d'énergie et l'unité (5) de centrale électrique.
